# EUROPEAN PATENT APPLICATION

(11) **EP 3 881 822 A1**
(43) Date of publication of application: **22.09.2021**
(21) Application number: 20209857.0
(22) Date of filing: 25.11.2020
(51) Int. Cl.: A61K 8/64, A61Q 5/00, A61Q 19/08, A61Q 7/00

(54) **COSMETIC METHOD FOR SKIN TREATMENT AND HAIR REGROWTH**

(30) Priority: 16.03.2020 IT 202000005542
(71) Applicant: Themis S.r.l., 25021 Bagnolo Mella (BS) (IT)
(72) Inventor: Beldrighi, Graziano, 25021 Bagnolo Mella (BS) (IT); Molinari, Matteo, 25021 Bagnolo Mella (BS) (IT)
(74) Representative: Leganza, Alessandro

(57) **Abstract**

The present invention relates to an efficient cosmetic method for skin treatment and hair regrowth. The dermocosmetic method includes the treatment of the skin with a cosmetic composition comprising NGF by mesoporation, electroporation combined with jontophoresis, jontophoresis, electroosmosis, polar osmosis, laser treatment, ultrasonic peeling or ultrasonic skin firming.

## Description

### Technical field

The present invention is placed in the field of cosmetic compositions and cosmetic, non-therapeutic, methods for the treatment of the skin, and, in particular, for treating the scalp to promote hair regrowth and for the treatment of wrinkles for decrease their depth.

### State of the Art

The beauty of people's skin has always attracted study by researchers and companies operating in cosmetics, with the development of preparations, lotions and creams that for decades have filled specialized shops as well as the aisles of large supermarkets and shopping centres. In particular, many lotions are known today that promise hair regrowth in individuals who have lost all or part of their hair, with more claimed rather than real effects. Even the industry of cosmetic compositions such as creams or gels dedicated to the beauty of the skin is nowadays particularly flourishing given the people's interest in appearing with a pleasant and youthful appearance.

However, the real effects manifested by most of the products on the market are rather limited, scarce or ephemeral, and, for example, baldness of people is treated by drugs or by invasive treatments such as by replanting the scalp with new hair.

Also the treatment of wrinkles in order to reduce their depth is mostly carried out with invasive methods, for example by subcutaneous injection of fillers, or however by means of micro-injections carried out with needles or by stripping the skin.

Therefore, there is a lack of a cosmetic composition and a cosmetic method of treatment of the skin which allows to improve the skin, for example, to reduce the depth of wrinkles or which allows to treat the scalp in order to promote hair regrowth, without invasive treatments, without using needles or skinning the skin and without therapeutic treatments or pharmacological treatments with drugs specifically designed and approved by the Health Authorities.

Cosmetic compositions, not being legally classified as drugs, could therefore be made available to the public through large-scale distribution, so that anyone could, for example, reduce the depth of wrinkles rather than promote hair regrowth without resorting to medical treatments or to drugs.

### Summary of the invetion

The problem addressed by the present invention is therefore that of providing a dermocosmetic method, non-therapeutic, which allows to treat the skin, and preferably, which allows to reduce the depth of wrinkles or which promotes hair regrowth, without the use of needles, scarring or other invasive treatments or methods of therapeutic or pharmacological treatment.

This problem is solved by the method and the cosmetic composition as outlined in the attached claims, the definitions of which form an integral part of the present description.

Further characteristics and advantages of the cosmetic method and composition of the present invention will emerge from the description of the preferred embodiments of the invention, provided as an indication of the invention.

### Brief description of the figures

Fig. 1 a) shows, a wrinkle of the skin of the face before the treatment with the method of the invention and the same wrinkle relieved after the treatment with the passage of time (respectively Fig. 2) b), c), d)) .
Fig. 2 shows, from top to bottom, the photo before the treatment with the method of the invention, and two photos (centre and bottom) which, after the treatment with the method of the invention, testify the effect of the invention with thick hair regrowth on the scalp.

### Detailed description of the invention

The problem addressed by the present invention is that of providing an efficient method of dermocosmetic treatment of the skin, in particular for the treatment of the scalp for hair regrowth or the treatment of wrinkles to decrease their depth, which allows to be carried out without the use of invasive treatments.

Therefore, an object of the present invention is a non-therapeutic method for the dermocosmetic treatment of the skin, comprising the following steps:
- making available a cosmetic composition comprising an amount of NGF comprised from 10⁻⁹% to 1% (w / w),
- applying said composition on the skin areas that require cosmetic treatment, causing the absorption of NGF into the skin by means suitable for promoting the penetration of NGF into the skin, where said means are selected from:
   mesoporation, electroporation combined with jontophoresis, jontophoresis, electroosmosis, polar osmosis, laser treatment, ultrasonic peeling, ultrasonic skin firming.

The values indicated above as (weight / weight) are parts by weight per cent with compared to the total weight of the formulation. Therefore from 10⁻⁹% (10⁻⁹ %) to 1% (weight / weight), means the percentage of the quantity of NGF compared to the total weight of the composition. The value of 10⁻⁹% means, for example, that on 100 g of composition there are 10⁻⁹ g of NGF or 1 nanogram of NGF on 100 grams of composition.

It has indeed been surprisingly found that the combination of a cosmetic composition comprising a certain amount of NGF with one of the following techniques which, by favoring the penetration of NGF into the skin, determine its absorption, allows to obtain the improvement effects of the skin, in particular the attenuation of the depth of wrinkles and the thick regrowth of hair, without using invasive methods or drugs. These techniques being the following: mesoporation, electroporation combined with jontophoresis, jontophoresis, electroosmosis, polar osmosis, laser treatment, ultrasonic peeling, or ultrasonic skin firming.

According to a preferred embodiment, therefore, the method for the dermocosmetic treatment of the skin is the treatment of the scalp for hair regrowth or the treatment of wrinkles to decrease their depth.

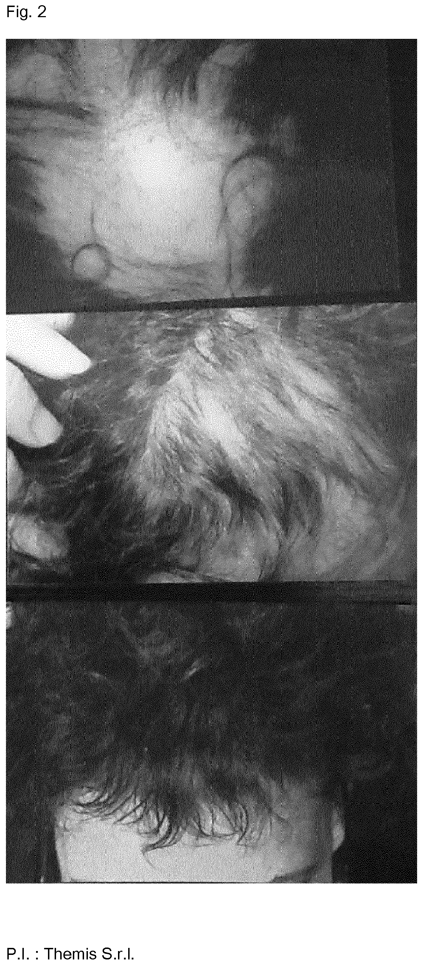

The comparison in Fig. 2 clearly shows the important effect of the present invention in the treatment of the scalp, by means of the method of the invention and therefore without invasive treatments such as by means of needles, scarring or scalp transplants.

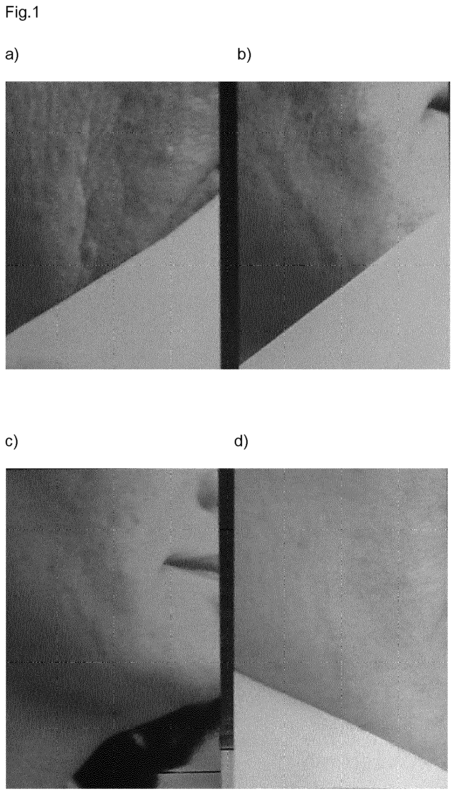

The comparison of the photos in Fig. 1 also shows the effect of the method of the present invention to reduce the depth of wrinkles.

NGF is the growth factor of nerve cells, in English Nerve Growth Factor, hence the abbreviation NGF, also called Neurotrophin 1 and having registration number 9061-61-4.

NGF is commercially available from Chemieliva Pharmaceuticals Co. Ltd, Chongqing (China) or from Sigma-Aldrich (USA); or it can be prepared according to the teachings of application WO2019207106A1.

The NGF included in the cosmetic composition of the invention can be of various origins, for example, of human origin, murine, etc. Preferably the NGF included in the cosmetic composition is of mouse origin, more preferably of mouse.

According to a preferred embodiment of the method according to the present invention, a cosmetic composition is made available comprising NGF-7S, i.e. Nerve Growth Factor (NGF) of murine origin, more preferably of mouse.

According to a preferred embodiment of the method according to the present invention, a cosmetic composition comprising NGF-7S, i.e. Nerve Growth Factor from the murine submaxillary gland, is made available, more preferably from the mouse submaxillary gland.

NGF-7S is classified with registration number 93928-24-6 and corresponds to a protein having an amino acid sequence composed of 241 amino acid residues.

NGF-7S is marketed by Chemieliva Pharmaceuticals Co. Ltd, referred to above or by Sigma-Aldrich with catalog number N0513, or it can be prepared according to the teachings of EP121338A1.

According to a more preferred embodiment of the method according to the present invention, a cosmetic composition comprising NGF-7S in the form of a lyophilized powder is made available (at Sigma-Aldrich with catalog number N0513).

According to a more preferred alternative embodiment of the method according to the present invention, a cosmetic composition is made available comprising NGF-7S in lyophilized form in physiological saline buffer at pH 7.2.

Mesoporation acts through the emission of electrical impulses that help to open small channels in the dermis, to carry the NGF in depth, even at high concentration, both on the skin and on the scalp.

Another technique is by means of a roller with an uneven surface and accentuated protuberances which favors the penetration of the NGF even in the deeper layers of the skin through the exertion of pressure. Said technique can be effectively applied also in combination with the other techniques mentioned above.

Another technique is electroporation, preferably with a spatula, combined with Jontophoresis. It is a combination of three technologies to treat skin areas with the composition including NGF.

With the spatula, preferably face spatula, it is possible to obtain a deep cleaning of the skin without the annoying wringing and in a pleasant way for the subject. It is indicated in the first phase of cleaning pre-treatments and / or preparation for transdermal delivery. The action of ultrasonic vibration allows the detachment "by pushing" of dead cells, also eliminating cosmetic residues and impurities deposited in the dilated pores. Vibration raises the crests of the corneocyte membranes and weakens the bonds of epidermal lipids, releasing a younger layer of corneocytes and making the skin finer and more luminous. Jontophoresis, on the other hand, represents the technological evolution of iontophoresis (or galvanic current) as a system of electro-conduction of ionic or ionizable active ingredients such as NGF. The current is modulated with polarity inversions making it more pleasant but equally effective in transporting the NGF by means of electrolytic dissociation.

Another technique, preferably used in combination with the others described above or below, is by means of humidity and / or occlusive substances and / or heat and / or massage.

Another technique is by means of ultrasounds. In particular, the ultrasonic peeling is a technique that promotes the removal of the upper layers of the epidermis, allowing the NGF to penetrate into the deeper layers of the skin and allowing the dermis to regenerate giving rise to a softer, brighter and smoother skin and to less deep wrinkles.

Main effects of ultrasonic peeling:
1. facilitates the absorption of NGF;
2. activates regenerative anti-inflammatory processes in case of acne and increases the elasticity of the tissues reducing the possible formation of post acne scars.
3. it relaxes the tissues thanks to the thermal and mechanical effect, thus obtaining the immediate relaxation of wrinkles.

Another technique is electroosmosis. Electroosmosis that can be carried out with the Cellular Electro-Plasty device which combines an electrical action (electrostimulation) with a mechanical action (skin pressing). Through skin pressing (touching and affixing gold electrodes to the skin) perfectly dosed micro currents are applied, which favor the skin renewal mechanisms.

Subsequently a cryothermic generator is applied to the skin to cause a thermal shock: vasoconstriction, followed by reflex vasodilation. This capillary gymnastics improves microcirculation favoring tissue oxygenation.

2. After stimulation, the skin is more receptive, and it is at this point that it is nourished with the composition comprising NGF, preferably through the application of Re-Plasty textures.

According to a preferred embodiment, the method for the dermocosmetic treatment of the skin is the treatment of the scalp for hair regrowth and wherein the cosmetic composition further comprises Minoxidril and / or Propecia.

Minoxidril and Propecia are two known active ingredients effective in the treatment of baldness.

Another technique is by means of a laser device which, acting directly on the cells, allows excellent absorption of NGF.

According to a preferred embodiment, the suitable means to favor the penetration of NGF into the skin is the laser treatment.

According to a preferred embodiment, the method for the dermocosmetic treatment of the skin is the treatment of the scalp for hair regrowth and wherein the cosmetic composition also comprises Minoxidril and / or Propecia and the means suitable for promoting the penetration of NGF in the skin is laser treatment.

In fact, another technique is by means of a laser device that promotes the hair regrowth effect by acting directly on the cells, which used, preferably, in combination with Minoxidil and / or Propecia, allows excellent absorption of NGF. The fractional laser treatments are called "fractional" because they only affect portions or "fractions" of the skin. Fractional laser treatments are preferred. Fractional lasers include Fraxel, which is a type of ablative CO₂ fractional laser, and Clear + Brilliant, which is a non-ablative CO₂ fractional laser. Basically, ablative fractional lasers such as Fraxel work by making small holes in the skin, which allows the NGF to penetrate deep into the skin, preferably into wrinkles or the scalp. This obviously increases the absorption in the NGF and therefore improves the skin and scalp.

Another technique is ultrasonic skin firming.

In ultrasonic skin firming, the ultrasound energy delivered, for example, through a treatment called "Ultherapy" employs sound waves that allow the skin to increase absorption of NGF. Ultrasonic skin firming, also called "ultrotherapy", can penetrate to different depths based on how the energy is focused.

Another technique is through a vibrating device which, through its vibrations, favors the enlargement of the pores allowing the NGF to penetrate deeply.

Another technique is polar osmosis. In this technique the NGF in limited concentrations, such as those of the invention, is applied dissolved or dispersed or emulsified in the composition. This medium works as a vehicle that accelerates or slows the transport of the substance through the stratum corneum.

The most common vehicles, that is, among the polar ones, water, and among the nonpolar ones, an oil, tend to accelerate the penetration of the NGF more with repulsive than attractive forces. Basically, simplifying a lot, with polar osmosis a hydro vehicle pushes a lipo active more while a lipo pushes a hydro active more.

According to a preferred embodiment, NGF is contained in said composition in a quantity comprised from 10-4% to 0.1% (weight / weight) of the total weight of the composition.

The composition comprising the NGF further and optionally comprises absorption promoters, substances that dilate the skin and make it more available to pass the molecules.

The composition comprising NGF can be found in very small pharmaceutical forms capable of slipping between one cell and another; among these are nanosomes and liquid crystals.

According to a preferred embodiment, the composition comprising the NGF is in the form of a microemulsion. In fact, when the composition comprising NGF is in the form of a microemulsion, good results are obtained, especially in hair regrowth.

An example of microemulsion is that of a composition comprising NGF in an amount comprised from 10⁻⁹% to 1% (weight / weight), eucalyptol, polysorbate 80, ethanol and water, more preferably in an amount comprised from 10⁻⁴% and 0.1% (weight / weight).

The composition comprising the NGF further and optionally comprises one more surfactants and / or solvents. Surfactants or solvents help NGF to penetrate the skin by solubilizing the lipid part and interfering with the metabolic activity that maintains barrier homeostasis. The surfactants in skin cleansers can also remove oils on the skin, leaving it more permeable to hydrophilic ingredients. Propylene glycol is a preferred solvent as it acts as an enhancer of NGF skin penetration.

According to a preferred embodiment, the composition comprising the NGF also comprises permeabilizers.

Permeants, permeators and permeabilizers are substances which increased permeability. The scientific literature talk of "penetration enhancer" "transcutaneous drug carrier" "percutaneous absorption enhancer". These are systems that increase skin permeability: therefore permeating, permeators or, more precisely, permeabilizers.

Both water and oil are solvents and theoretically act by permeating the skin barrier. The simple skin hydration facilitates the penetration of various substances even if the thickness of the horny layer increases. Prolonging the exposure of the stratum corneum to water for many hours it is induced desquamation and disorganization of skin lipids.

But to increase the penetration of NGF it can act with solvents which, in a short time, compromise the integrity of the barrier. Among the many mechanisms hypothesized to explain the greater transcutaneous penetration, the one with the greatest effect is the permeabilizers, substances or mixtures capable of thinning skin lipids. In practice, the permeabilizer would interfere with the lipid structures of the skin, disorganizing and / or interposing them.

Solvents, cosolvents, surfactants and hydrotropics would easily reach this effect.

The more rapidly the vehicle has the ability to diffuse and thin the skin lipids, the less the stratum corneum will oppose the diffusion of any active ingredients contained in the cosmetic.

The more "potent" solvents increase the penetration of both lipophilic and hydrophilic substances. A vehicle where both NGF and skin lipids dissolve easily can significantly increase the diffusivity of NGF through the stratum corneum. The permeabilizer system is actually usually relatively complex, where the action of specific permeabilizers depends on the concentrations in a medium and on the synergies with other solvents and permeabilizers.

The permeabilizers that can optionally be included in the composition of NGF of the invention, even in any combination between them, are:
- alcohols: both short ethanyl alcohol, methanol, isopropyl alcohol, and longer chain such as decyl alcohol, hexanol, octanol, myristyl alcohol;
- dimethyl solvents such as dimethyl sulfoxide, acetone, or dimethyl isosorbide;
- glycols: propylene glycol with also its esters, longer chain glycols such as butylene glycol, hexylene glycol;
- fatty acids: preferably having chains composed of 8-12 carbon atoms;
- pyrrolidones: methyl pyrrolidone, 2-pyrrolidone;
- surfactants: transcutaneous surfactant permeabilizers of each group, anionic, cationic, non-ionic, amphoteric, etc .;
- terpenes: limonene, menthol, cineole, linalool;
- amides, in particular those with the nitrogen atom that can act as an acceptor, preferably laurocaprame or niacinamide;
- urea;
- Hydrotropes, cosolvents and eutectic mixtures;
- complexing or encapsulating: i.e. systems that "wrap" the NGF in permeabilizing structures. In some ways, emulsions are already systems that can increase the penetration of NGF, but encapsulations of smaller dimensions than the typical "droplets" dispersed in an emulsion can be organized. Cyclodextrins or phospholipids with which liposomes are formed can act in a similar way, but also waxy solid lipids in which the NGF that it is wanted to penetrate is pre-dispersed.

To increase the diffusion, it is possible to use absorption promoters, the "enhancers", agents able to reduce the barrier efficiency with chemical or physical mechanisms, increasing the diffusibility of the substance inside the barrier by interaction with intracellular keratin, with desmosomes, with intercorneocyte lipids.

To increase diffusion, excipients such as ethanol and propylene glycol can be used, which, thanks to their solvent properties, positively influence the solubility of NGF in the skin.

According to a preferred embodiment, in the composition comprising the NGF, the NGF is found in microinscapsulated form.

Microencapsulation allows NGF to be released into the skin for longer periods of time.

The microencapsulated NGF allows a better penetration of the same into the skin, partly because the ingredients have a greater amount of time to pass through the skin and partly because the NGF above the skin does not degrade.

According to a preferred embodiment, the means suitable for promoting the penetration of NGF into the skin is iontophoresis or electroporation, applied on the composition comprising NGF and in one or more of the following cases and their combinations:
- the dermocosmetic treatment of the skin is the treatment of the scalp for hair regrowth and where the cosmetic composition further includes Minoxidril and / or Propecia,
- in the composition including NGF, NGF is found in microinscapsulated form,
- the composition comprising the NGF further includes permeabilizers,
- the composition comprising the NGF is in the form of a microemulsion,
- NGF is contained in said composition in a quantity comprised from 10⁻⁴% to 0.1% (weight / weight) of the total weight of the composition.

Iontophoresis (also known as "iontophoresis" and "medicinal dielectrolysis") is a specific technique that falls within the sphere of electrotherapies. There are two types of electrotherapy treatments:
- continuous current: iontophoresis and galvanotherapy;
- alternating current (low, medium and high frequency):
   electrolipolysis, TENS, Kotz currents, etc.

Technically, Iontophoresis consists in the administration of NGF transcutaneously through the exploitation of a continuous current that is produced by a specific instrument.

Iontophoresis has the primary advantage of being able to introduce NGF into the patient's body, without necessarily combining it with other substances in order to be able to carry it without causing damage to organs and tissues.

As mentioned above, the composition comprising the NGF can be applied through Electroporation, i.e. a sudden electric discharge. The discharge simultaneously opens the plasma membrane of cells in numerous places, allowing DNA molecules to penetrate. It is possible to optimize the electroporation parameters for the different types of cell lines, by varying the intensity of the electric field and the duration of the treatment. Face electroporation is performed with a special handpiece which, with the help of a microcurrent electric field, makes the NGF, connected to the handpiece, penetrate inside the skin, greatly increasing its effectiveness. The electric field allows the NGF to be attracted to the inside of the tissue, or, the application of electro-magnetic fields that create a perturbation of the skin for a few fractions of a second and this makes it permeable to NGF. It is a safe and non-invasive method that uses tested frequencies and voltages. This technique is used using a machine that, for example, has a handpiece with two fixed electrodes inserted at a fixed distance that are repeatedly passed on the areas of the skin to be treated (carefully cleansed) on which the composition including the NGF is spread in advance, preferably in the form of a gel. It is necessary to pay attention that the technique used is not actually an iontophoresis, in this case we are in the presence of an ionization chamber and more evidently, the two electrodes are separated and at variable distance with consequent risk of potential oscillations and possible irritation cutaneous.

In the case of electroporation, pores are opened between the cells, in the case of iontophoresis the substances are conveyed through the passage of current and this implies that they are previously loaded into the ionization chamber.

Another object is the use of the cosmetic composition, including the preferred or alternative embodiments described above, for the dermocosmetic treatment of the skin by means suitable for promoting the penetration of NGF into the skin, selected from mesoporation, electroporation combined with jontophoresis, jontophoresis, electroosmosis, polar osmosis, laser treatment, ultrasonic peeling, ultrasonic skin firming.

According to a preferred embodiment, the use in which the cosmetic composition comprises an amount of NGF comprised from 10⁻⁹% to 1% (weight / weight), or, more preferably, from 10⁻⁴% to 0.1% (weight/ weight).

## Claims

1. Non-therapeutic method for skin dermocosmetic treatment, comprising the following steps:
- providing a cosmetic composition comprising an amount of NGF ranging from 10⁻⁹% to 1% (weight / weight),
- apply said composition on the skin areas that need cosmetic treatment, causing the absorption of NGF into the skin by means suitable to promote the penetration of NGF into the skin, where said means are chosen from:
mesoporation, electroporation combined with jontophoresis, jontophoresis, electrosmosis, polar osmosis, laser treatment, ultrasonic peeling, ultrasonic skin firming.

2. Method according to the claim 1, wherein the dermocosmetic treatment of the skin is the treatment of the scalp for the regrowth of the hair or the treatment of wrinkles to decrease their depth.

3. Method according to claim 1, wherein dermocosmetic treatment of the skin is the treatment of the scalp for hair regrowth and where cosmetic composition further comprises Minoxidril and / or Propecia.

4. Method according to any one of claims from 1 to 3, wherein the suitable means for promoting the penetration of NGF into the skin is laser treatment.

5. Method according to any one of claims from 1 to 4, wherein in the composition comprising the NGF, the NGF is in a micro-encapsulated form.

6. Method according to any one of claims from 1 to 5, wherein the composition comprising the NGF also includes permeabilizers.

7. Method according to any one of claims 1 to 6, wherein the composition comprising the NGF is in the form of a microemulsion.

8. Method according to any one of claims from 1 to 7, wherein the NGF is contained in said composition in an amount ranging from 10⁻⁴% to 0.1 % (weight / weight).

9. Method according to any one of claims 3, from 5 to 8, wherein the means suitable for promoting the penetration of NGF into the skin is iontophoresis or electroporation.

10. Use of the cosmetic composition according to any one of the claims 1, 3, and from 5 to 8, for the dermocosmetic treatment of the skin by means suitable for promoting the penetration of NGF into the skin, chosen from mesoporation, electroporation combined with iontophoresis, iontophoresis, electrosmosis, polar osmosis, laser treatment, ultrasonic peeling, ultrasonic skin firming.
